# EUROPÄISCHE PATENTSCHRIFT

(11) **EP 0 680 948 B1**
(45) Veröffentlichungstag und Bekanntmachung des Hinweises auf die Patenterteilung: **08.04.1998**
(21) Anmeldenummer: 95106329.6
(22) Anmeldetag: 27.04.1995
(51) Int. Cl.: C07C 233/47, C07C 231/08

(54) **Verfahren zur Herstellung von N-Acylglycinderivaten**
Process for preparing N-acylglycin derivatives
Procédé pour la préparation de dérivés de glycine N-acylée

(30) Priorität: 02.05.1994 DE 4415312
(43) Veröffentlichungstag der Anmeldung: 08.11.1995
(73) Patentinhaber: HOECHST AKTIENGESELLSCHAFT, 65929 Frankfurt am Main (DE)
(72) Erfinder: Beller, Matthias, Dr., D-65510 Idstein (DE); Fischer, Hartmut, Dr., D-65719 Hofheim (DE); Gerdau, Thomas, Dr., D-65817 Eppstein (DE); Gross, Peter, D-65451 Kelsterbach (DE)

(56) Entgegenhaltungen:
- DE-C- 849 554
- FR-A- 862 365
- GB-A- 2 252 770
- US-A- 4 891 442
- US-A- 4 918 222
- C. FERRI, "Reaktionen der organischen Synthese", 1978, GEORG THIEME VERLAG, Stuttgart, Seite 293 * Abschnitt 12 *

## Beschreibung

Die vorliegende Erfindung betrifft ein neues, verbessertes Verfahren zur Herstellung von N-Acyl-α-aminosäurederivaten, insbesondere N-Acylsarkosinen, durch Umsetzung von Carbonsäureamiden mit Aldehyden und CO unter Säurekatalyse durch Cobaltcarbonylverbindungen.

N-Acyl-α-aminosäurederivate, insbesondere die N-Acylsarkosine haben technische Bedeutung als Bestandteil von Tensiden, Seifen und Emulgatoren.

Das heute in der Technik benutzte Verfahren zur Synthese derartiger Verbindungen besteht darin, Fettsäurechloride mit dem Natriumsalz des Glycins oder Sarcosins in einer klassischen Schotten-Baumann-Reaktion umzusetzen. Das dabei zwingend anfallende Salz sowie die Nutzung von Chlorierungsmitteln wie Phosgen oder Phosphortrichlorid zur Herstellung der Fettsäurechloride sind unter ökologischen Aspekten sehr nachteilig (J. Am. Chem. Soc. 78, 172, (1956)).

Ein ökologisch verbessertes Verfahren besteht in der Umsetzung von Fettsäureamiden, die durch Aminolyse direkt aus natürlichen Fettsäuren oder Fetten zugänglich sind, mit Formaldehyd und CO in Gegenwart eines Katalysators. Diese als Amidocarbonylierung bezeichnete Reaktion wurde zuerst von Wakamatsu beschrieben in Chem. Commun. 1971, 1540 und in DE 2.115.985. Danach wurde aus Acetamid, Paraformaldehyd und CO N-Acetylglycin jedoch lediglich in 26 % Ausbeute erhalten.

Weitere Varianten sind z.B. in der EP 170.830 und EP 197.659 beschrieben. Hier wird die Amidocarbonylierung von Paraformaldehyd mit Acetamid zu Acetylglycin aufgeführt, wobei Promotoren wie Nitrile, Sulfoxide oder Phosphane die Selektivitäten erhöhen und die Rückführung des Katalysators verbessern sollen. Das N-Acetylglycin wird jedoch selbst unter optimierten Bedingungen im besten Fall lediglich in 70 % Ausbeute gewonnen.

In der Literatur wird auch beschrieben, daß am N-Atom alkylierte Amide deutlich schlechtere Ausbeuten an den N-Alkyl-acylaminosäuren liefern als vergleichbare primäre Amide (P. Magnus, M. Slater, Tetrahedron Lett. 1987, 28, 2829).

In J. Org. Chem. 147, 99 (1991) ist die Herstellung von N-Acylsarcosin durch Carbonylierung von N-Methyllaurylamid bei einem CO + H₂ (3:1)-Druck von über 200 bar beschrieben. Bei diesem Verfahren wird das gewünschte Produkt nur sehr verunreinigt erhalten.

Die GB 2 252 770 beschreibt eine Einstufensynthese von N-Acylaminosäuren durch Umsetzung eines Carbonsäureamids mit einem Aldehyd und CO in Gegenwart eines Metallkatalysators und einer Säure als Cokatalysator.

Bei diesem Verfahren wird das Carbonsäureamid in sehr hohem Überschuß bezogen auf den Aldehyd (1.78 zu 1.0) eingesetzt, so daß dieses Verfahren bezogen auf das eingesetzte Acetamid nur mäßige Ausbeuten liefert. Außerdem ist hierdurch das Produkt mit mindestens 80 % Edukt verunreinigt, was das Verfahren für technische Anwendung unbrauchbar macht.

Alle beschriebenen Verfahren laufen mit nur unzureichenden Umsätzen und Selektivititäten ab, liefern verunreinigte Produkte oder benötigten sehr hohe CO-Drucke.

DE-A-364 204 beschreibt lediglich ein Verfahren zur Herstellung von N-Acylglycinen ausgehend von N-Hydroxymethylamiden mit Kohlenmonoxid und Wasserstoff in Gegenwart einer Cobaltcarbonylverbindung in Wasser oder einem inerten, Wasser enthaltenden Lösemittel als Reaktionsmedium.

Nachteilig an diesem Verfahren ist die Umsetzung in Wasser oder in stark wasserhaltigen Lösemitteln.

Es bestand somit ein großer Bedarf nach einem Verfahren, das N-Acyl-α-aminosäurederivate, insbesondere N-Acylsarkosine in hoher Ausbeute und Reinheit auf technisch leicht realisierbare Weise zugänglich macht.

Diese Aufgabe wird gelöst durch ein Verfahren zur Herstellung von Acylglycinderivaten der allgemeinen Formel (I) 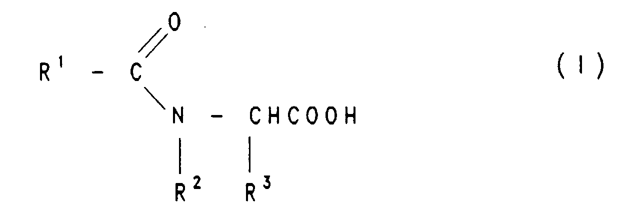 worin
- R¹: Wasserstoff, ein gesättigter, geradkettiger, verzweigter oder zyklischer (C₁-C₂₆)Alkylrest, ein ein- oder mehrfach ungesättigter, geradkettiger, verzweigter oder zyklischer (C₂-C₂₄)Alkenylrest, ein (C₆-C₁₈)Arylrest, ein (C₁-C₁₀)Alkyl-(C₆-C₁₈)Arylrest oder ein gegebenenfalls mehrfach ungesättigter (C₂-C₁₀)Alkenyl-(C₆-C₁₈)Arylrest,
- R²: Wasserstoff, ein gesättigter, geradkettiger, verzweigter oder zyklischer (C₁-C₂₆)Alkylrest, ein ein- oder mehrfach ungesättigter, geradkettiger, verzweigter oder zyklischer (C₂-C₂₃)Alkenylrest, ein (C₆-C₁₈)Arylrest, ein (C₁-C₁₀)Alkyl-(C₆-C₁₈)-Arylrest oder ein gegebenenfalls mehrfach ungesättiger (C₂-C₁₀)Alkenyl-(C₆-C₁₈)Arylrest
und
- R³: Wasserstoff, ein gesättigter, geradkettiger, verzweigter oder zyklischer (C₁-C₁₀)Alkylrest, ein ein- oder mehrfach ungesättigter, geradkettiger, verzweigter oder zyklischer (C₂-C₁₀)Alkenylrest, ein (C₆-C₁₈)-Arylrest, ein (C₁-C₁₀)Alkyl-(C₆-C₁₈)Arylrest oder ein gegebenenfalls mehrfach ungesättigter (C₂-C₁₀)Alkenyl-(C₆-C₁₈)-Arylrest bedeuten,
das dadurch gekennzeichnet ist, daß man ein Carbonsäureamid der allgemeinen Formel (II) 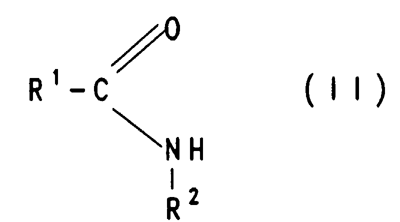 worin R¹ und R² die oben angegebene Bedeutung besitzen mit einem Aldehyd der Formel R³-CHO in Gegenwart eines Lösungsmittels und einer Säure zu einem Acylaminomethylol der Formel (III) 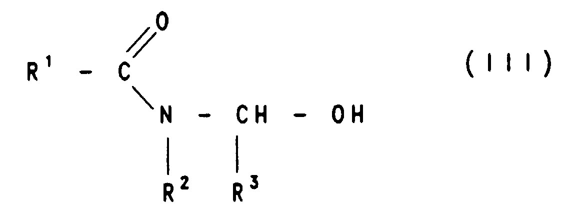 umsetzt und dieses anschließend nach Zusatz eines Cobaltcarbonylkatalysators und einer Säure als Cokatalysator bei einer Temperatur von 20 bis 150°C und einem CO-Druck von 1 bis 150 bar carbonyliert.

Bevorzugt bedeuten:
- R¹: einen gesättigten, geradkettigen oder verzweigten (C₈-C₂₄)Alkylrest, insbesondere (C₁₀-C₁₈)Alkylrest, einen ein- oder mehrfach ungesättigten, geradkettigen oder verzweigten (C₈-C₂₄)Alkenylrest, insbesondere (C₁₀-C₁₈)Alkenylrest,
- R²: Wasserstoff, einen gesättigten, geradkettigen oder verzweigten (C₁-C₈)Alkylrest, insbesondere (C₁-C₄)Alkylrest oder einen ein- oder mehrfach ungesättigten, geradkettigen oder verzweigten (C₂-C₈)Alkenylrest,
- R³: Wasserstoff, einen gesättigten, geradkettigen oder verzweigten (C₁-C₆)Alkylrest oder einen ein- oder mehrfach ungesättigten, geradkettigen oder verzweigten (C₂-C₆)Alkenylrest.

Die Reste R¹, R² und R³ können gegebenenfalls substituiert sein. Geeignete Substituenten sind die Hydroxylgruppe, (C₁-C₁₀)Alkoxyreste und Halogenatome.

Geeignete Amide sind z.B. Formamid, Acetamid, N-Methylacetamid, Propionamid, Butyramid, Acrylamid, N-Methylformamid, N-Methylbenzamid, Benzamid und Crotonamid.

Als Ausgangsstoffe für das erfindungsgemäße Verfahren besonders geeignete Amide sind Amide und N-Alkylamide, insbesondere N-Methylamide der geradkettigen oder verzweigten, gesättigten oder ungesättigten Carbonsäuren mit 8 bis 24 C-Atomen. Im einzelnen sind zu nennen: Octansäureamid, 2-Ethylhexansäureamid, Decansäureamid, Laurinsäureamid, Palmitinsäureamid, Stearinsäureamid, Ölsäureamid, Linolsäureamid, Linolensäureamid, Gadoleinsäureamid und Nervonsäureamid.

Besonders bevorzugte Amide sind die N-Methylamide von natürlichen Fettsäuren, wie Laurinsäure, Palmitinsäure, Stearinsäure und Ölsäure.

Die Amide der Formel (II) können als Reinsubstanzen oder als Gemische eingesetzt werden. Geeignete Gemische stellen die natürlich vorkommenden Fette dar, z.B. Kokosnuß-, Babassu-, Palmkern-, Palm-, Oliven-, Ricinus-, Erdnuß-, Raps-, Rinder-, Schweine-, Walfischfett bzw. -öl (zur Zusammensetzung dieser Fette s. Fieser und Fieser, Organische Chemie, Verlag Chemie 1972, Seite 1208).

Geeignete Aldehyde sind z.B. Formaldehyd, Acetaldehyd, Propionaldehyd, Butyraldehyd, Isobutyraldehyd, Furfural, Crotonaldehyd, Acrolein, Benzaldehyd, Phenylacetaldehyd, 2,4-Dihydroxyphenylacetaldehyd und α-Acetoxypropionaldehyd. Ebenfalls geeignet sind Substanzen, welche unter den genannten Reaktionsbedingungen einen Aldehyd bilden können, z.B. Aldehydoligomere, wie Paraformaldehyd und Paraldehyd. Es hat sich in vielen Fällen bewährt, Formaldehyd in Form von Paraformaldehyd einzusetzen.

Das erfindungsgemäße Verfahren wird in zwei Stufen ausgeführt. In der ersten Stufe wird zunächst aus dem Aldehyd und dem Carbonsäureamid das Acylaminomethylol der Formel (III) gebildet, welches im zweiten Schritt mit CO zum Endprodukt umgesetzt wird. Diese zweistufige Verfahrensweise ermöglicht überraschenderweise eine deutliche Steigerung von Umsatz und Selektivität in jeder Stufe, so daß für den Gesamtprozeß Umsätze von 100 % des Carbonsäureamids bei Selektivitäten von 98 % zum N-Acyl-α-aminosäurederivat erreicht werden, d.h. auch die Ausbeuten an Zielprodukt 98 % betragen.

Besonders günstig an dem erfindungsgemäßen Verfahren ist, daß schon äquimolare Mengen Aldehyd hohe Ausbeuten ergeben und so Produkte erhalten werden können, die nicht durch Aldehyd verunreinigt sind. Es ist jedoch auch möglich mit Aldehydüberschüssen zu arbeiten.

Es hat sich als vorteilhaft erwiesen, 70 bis 200 Mol-%, insbesondere 100 bis 140 Mol-%, bevorzugt 100 bis 120 Mol-% Aldehyd, bezogen auf das Carbonsäureamid einzusetzen.

Die Addition des Aldehyds an das Carbonsäureamid in Gegenwart einer Säure erfolgt beim Erwärmen in Lösung. Als Säuren können neben organischen Säuren, wie Toluolsulfonsäure, Hexafluorpropansulfonsäure oder Trifluoressigsäure und anorganischen Säuren, wie Schwefelsäure, Phosphorsäure auch Ionenaustauscherharze verwendet werden.

Sehr gut geeignet ist Schwefelsäure. Die in das Reaktionssystem eingebrachte Säure kann in der Lösung des gebildeten Acyl-aminomethylols verbleiben, ohne daß die nachfolgende Carbonylierung dadurch gestört wird.

Es hat sich in vielen Fällen bewährt mit Säurekonzentrationen von 0,2 bis 5 Mol-%, insbesondere 0,5 bis 4 Mol-%, bevorzugt 1,0 bis 2,5 Mol-%, bezogen auf das Amid, zu arbeiten.

Die Reaktionsfolge wird zweckmäßig in einem polar-aprotischen Lösemittel durchgeführt, wie z.B. Tetrahydrofuran, Glykoldimethylether, Methyl-t-butylether, Diglykoldimethylether, Dimethylformamid, Dimethylacetamid oder Acetonitril. Als besonders geeignet haben sich Tetrahydrofuran, Glykoldimethylether (Glyme) und Methyl-t-butylether erwiesen. In der ersten Stufe wird in einem Rührreaktor bei Normaldruck das Carbonsäureamid mit dem Aldehyd umgesetzt. Diese Reaktion läuft bei 65 bis 120°C innerhalb von 10 bis 60 min ab.

Bei der Durchführung des erfindungsgemäßen Verfahrens ist die in dem Reaktionsansatz vorhandene bzw. sich bildende Wassermenge möglichst gering zu halten. Angestrebt werden hierbei Wassermengen von bis zu 2 Gew.-%, üblicherweise zwischen 0,1 bis 1 Gew.-%, bezogen auf den Reaktionsansatz. Aus diesem Grund wird der Einsatz wasserfreier Lösungsmittel bevorzugt. Denkbar ist der Einsatz sogenannter technischer Lösungsmittel, die im Hinblick auf den Wassergehalt den obigen Anforderungen genügen müssen.

Man erhält dann klare Lösungen, aus denen auch bei längerem Stehen (mehrere Tage) bei Zimmertemperatur kein Feststoff auskristallisiert. Diese Lösungen werden aus Gründen der Reaktionsführung unmittelbar nach ihrer Herstellung zur Carbonylierung eingesetzt. Überraschenderweise sind die erhaltenen Lösungen relativ stabil, so daß die Weiterverarbeitung auch nach einer gewissen Aufbewahrungszeit erfolgen kann.

Es ist ein wichtiger technischer Vorteil des Verfahrens, daß man diese Lösungen kontinuierlich über eine Druckdosierpumpe dem Carbonylierungsreaktor zuführen kann, wodurch die exotherme Reaktion gut zu steuern ist.

Die Carbonylierung des Zwischenproduktes der Formel (III) zu dem Endprodukt der Formel (I) wird mit Kohlenmonoxid von 1 bis 150 bar in einem geeigneten Reaktor bei Temperaturen von 20 bis 150°C, insbesondere bei 25 bis 100°C, bevorzugt bei 30 bis 70°C unter Katalyse durch Cobaltcarbonylverbindungen durchgeführt. Kohlenmonoxid wird zweckmäßig als reines Gas angewendet, weil das Restgas dann ohne weiteres im Kreis gefahren werden kann. Das eingesetzte Kohlenmonoxid kann auch eine begrenzte Menge Wasserstoff enthalten. Selbst wenn das eingesetzte Kohlenmonoxid mit anderen Gasen, z.B. Stickstoff, Methan, Kohlendioxid, welche Wassergas gewöhnlich enthält, verunreinigt ist, hat dies keinen nachteiligen Einfluß auf die Reaktion. Der anzuwendende Druck beträgt mindestens 1 bar und muß 100 bar nicht übersteigen. Bei geeigneter Gestaltung des Reaktors zum wirkungsvollen Gaseintrag in die Lösung, z.B. in einem Rührreaktor mit Begasungsrührer oder in einer Blasensäule, kann der CO-Druck ohne weiteres auf weniger als 50 bar gesenkt werden. Das Verfahren wird deshalb bevorzugt bei 1 bis 50, besonders bevorzugt bei 3 bis 20 bar CO-Druck ausgeführt. Es können auch CO-haltige Gasgemische, z.B. Synthesegas CO + H₂ im Verhältnis 1:1 eingesetzt werden. Im Restgas wird dann allerdings Wasserstoff angereichert, was die Kreislauffahrweise kompliziert und den Gesamtdruck des Reaktionssystems erhöht.

Die Carbonylierung wird durch Cobaltcarbonyl katalysiert. Dieses kann als festes Co₂(CO)₈ zu den Lösungen des Methylols (III) zugegeben, gelöst und dann in den Carbonylierungsreaktor eingebracht werden. Das Cobaltcarbonyl kann jedoch auch in großer Menge auf Vorrat in einem separaten Druckreaktor aus einer geeigneten Cobalt(II)-verbindung wie z.B. Cobalt(II)acetat, basischem Cobalt(II)carbonat oder Cobalt(II)-ethylhexanoat und CO, gegebenenfalls unter Zusatz von H₂, im gleichen Lösemittel wie für die Methylol-Stufe benutzt, gebildet werden. Von dieser Cobaltcarbonyl-Lösung wird dann ein Anteil in den Carbonylierungsreaktor zur Lösung des Methylols III gegeben. Die Herstellung und Vorratshaltung des Co₂(CO)₈ in Lösung hat den Vorteil, daß man den luftempfindlichen toxischen Stoff nicht als Feststoff handhaben muß; die Lösungen können durch Überlagerung mit CO stabilisiert werden. Die Menge an zugegebenem Co₂(CO)₈ ist so zu bemessen, daß die Reaktionsmischung 0,1 bis 5,0, bevorzugt 0,6 bis 2,0 Mol-% Co, bezogen auf das in Stufe 1 eingesetzte Carbonsäureamid enthält. Mit der bevorzugten Katalysatorkonzentration springt die Reaktion bei ca. 20°C an, erkennbar an der CO-Aufnahme. Bei 70°C Reaktionstemperatur wird die Reaktion so schnell, daß Raum/Zeit-Ausbeuten von 300 g/l.h erreicht und überschritten werden. Während der Carbonylierung ist auf einen hinreichend intensiven Gaseintrag in die Lösung zu achten, um einen quantitativen Umsatz zu erreichen.

Nach Abklingen der Reaktion, die in Abhängigkeit vom angewendeten CO-Druck 0.5 bis 2.0 h erfordert, wird abgekühlt und das überschüssige Gas entspannt. Aus dem Reaktor entnimmt man eine klare, gelb bis braun gefärbte Lösung, aus der zunächst der homogen gelöste Katalysator entfernt werden muß. Dies wird in üblicher Weise durchgeführt, indem die Cobaltcarbonylverbindungen durch Einblasen von Luft oxidativ zerstört werden und das sich dabei bildende zweiwertige Cobalt als schwerlösliches Salz, z.B. Oxalat, Phosphat, Sulfat oder Carbonat ausgefällt wird. Dieses wird abfiltriert. Die resultierende Lösung ist höchstens schwach gelb gefärbt und enthält das Zielprodukt in 98 %iger Ausbeute. Die Isolierung und Reinigung gelingt einfach, so daß bei dieser Operation nur minimale Ausbeute-Verluste auftreten. Das Lösemittel wird in einem Dünnschichtverdampfer destillativ abgetrennt; das Destillat kann ohne Einschränkung in den Prozeß zurückgeführt werden. Das ablaufende Konzentrat, bestehend aus geschmolzenem Rohprodukt, wird in heißes Wasser eingetragen, gut dispergiert und durch Abkühlen kristallisiert. Durch Filtration isoliert man ein weißes, wasserfeuchtes Produkt, das sofort für die meisten Anwendungen geeignet ist. Sorgfältige Bestimmung des Wassergehaltes verbunden mit HPLC-Analysen sowohl des Feucht- als auch des Trockenproduktes belegen, daß die Ausbeuten an Zielprodukt, bezogen auf das jeweils eingesetzte Carbonsäureamid, 94 bis 98 % d.Th. betragen.

Das erfindungsgemäße Verfahren liefert N-Acyl-α-aminosäurederivate, insbesondere N-Acylglycine und N-Acylsarkosine in sehr guter Reinheit in praktisch quantitativer Ausbeute, ohne daß Nebenprodukte anfallen oder eine aufwendige Aufarbeitung oder Nachreinigung erforderlich wäre.

Das erfindungsgemäße Verfahren eignet sich vorteilhaft zur Herstellung von N-Acylsarkosinen auf Basis von N-Acylamiden langkettiger gesättigter bzw. ungesättigter Fettsäuren.

Die nachstehenden Beispiele sollen das Verfahren erläutern.

### Beispiel 1

### Herstellung von Lauroylsarcosin

213 g (1 Mol) Laurinsäure-N-methylamid und
34 g Paraformaldehyd (95 %ig ≙ 1,08 Mol) werden in
350 ml Dimethoxyethan (glyme) suspendiert und mit
2 g (0,02 Mol) H₂SO₄ versetzt.

1) Diese Mischung wird unter Rühren zum Sieden erhitzt (Kp = 84°) und 5 bis 10 min bei dieser Temperatur gehalten. Dabei lösen sich die Feststoffe weitgehend auf. Man läßt auf ca. 60°C abkühlen und filtriert die noch warme, leicht getrübte Lösung.
   Man erhält eine klare Lösung, die bei Zimmertemperatur in geschlossenen Gefäßen ohne Zersetzung aufbewahrt werden kann. In offenen Gefäßen verliert die Lösung allerdings langsam gasförmigen Formaldehyd und nach einigen Tagen beginnt Laurinsäure-N-methylamid auszukristallisieren.
2) Die Lösung des Additionsproduktes aus Laurinsäure-N-methylamid und Formaldehyd wird mit 2,02 g (5,85 mMol) Co₂(CO)₈ versetzt (entsprechend 11,7 mMol CO oder 1,17 %) und in einen 1l-Autoklaven eingefüllt. Es wird 20 bar CO aufgedrückt und auf 70°C erwärmt. Das Einsetzen der Reaktion ist am fallenden Druck zu erkennen; es wird dann CO nachgegeben, um den Druck im Reaktor zu halten. Der größte Teil des Gases wird innerhalb von 30 bis 60 min aufgenommen; um den Umsatz sicher auf 100 % zu bringen, wird 1 h nachgerührt. Der Reaktor wird abgekühlt, das überschüssige Gas entspannt und die gelbe, klare Produktlösung entnommen.

Die cobaltcarbonylhaltige Lösung wird mit 2 g (22 mMol) Oxalsäure versetzt und unter gutem Rühren wird Luft eingeleitet. Nach 1 h ist die Ausfällung des Cobaltoxalats komplett. Dieses wird abfiltriert. Anschließend wird in einem Dünnschichtverdampfer (Öltemperatur 140 bis 150°C) aus der Lösung das Glyme entfernt. Das als Konzentrat abfließende, geschmolzene Rohprodukt wird in 1 l 60-80°C warmem Wasser dispergiert (emulgiert), um Reste von Lösemittel, Formaldehyd und der Säure aus Reaktionsstufe 1, zu entfernen. Die Emulsion wird unter Rühren langsam abgekühlt, bei 15 bis 5°C kristallisiert das Lauroylsarcosin. Es wird abgesaugt, mit Wasser nachgewaschen und trockengepreßt.
Ausbeute wasserfeuchtes Produkt: 451,7 g
Die Feuchtebestimmung ergab einen Wasseranteil von 41.2 % = 186 g H₂O.
Ausbeute an Lauroylsarcosin = 265.6 ≙ 98 % d.Th.
Das trockene Produkt hat einen Fp = 49 bis 50°C.
HPLC und H-NMR bestätigen eine hohe Reinheit > 99.7 %.

### Beispiele 2 bis 9

Diese Beispiele wurden analog zu Beispiel 1 mit anderen Ausgangsstoffen durchgeführt, teilweise in verkleinertem Maßstab. Stoffmengen und Ergebnisse sind in Tabelle 1 zusammengefaßt. Die 0,2 Mol-Ansätze wurden in einem 200 ml-Autoklaven durchgeführt, wobei der CO-Druck auf 50 bar erhöht war.

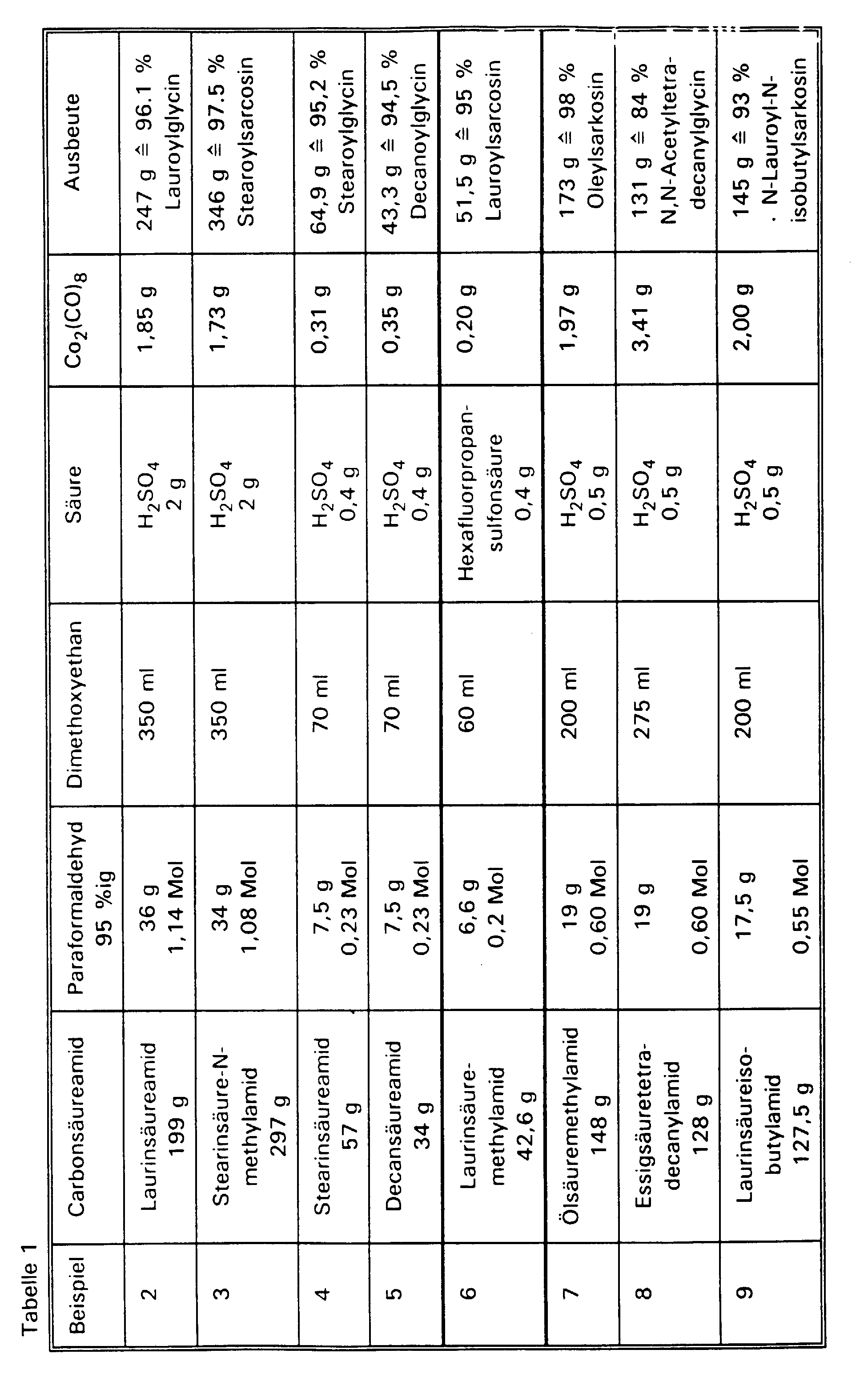

### Beispiel 10

### Herstellung von N-Lauroyl-1-propyl-sarkosin

21,3 g (0,1 Mol) Laurinsäure-N-methylamid und
8,8 g Butyraldehyd (0,12 Mol) werden in
35 ml Ethylacetat suspendiert und mit
0,3 g (1,3 mMol) Hexafluorpropansulfonsäure versetzt.

1) Diese Mischung wird unter Rühren im Autoklaven auf 95°C erhitzt und 5 bis 10 min bei dieser Temperatur gehalten. Man läßt anschließend auf Raumtemperatur abkühlen.
2) Die Lösung des Additionsproduktes aus Laurinsäure-N-methylamid und Butyraldehyd wird mit 0,35 g (1,02 mMol) Co₂(CO)₈ versetzt. Es wird 50 bar CO aufgedrückt und auf 70°C erwärmt. Das Einsetzen der Reaktion ist am fallenden Druck zu erkennen; es wird dann CO nachgegeben, um den Druck im Reaktor zu halten. Der größte Teil des Gases wird innerhalb von 60 min aufgenommen; um den Umsatz sicher auf 100 % zu bringen, wird 2 h nachgerührt. Der Reaktor wird abgekühlt, das überschüssige Gas entspannt und die gelbe, klare Produktlösung entnommen.

Die cobaltcarbonylhaltige Lösung wird mit 0,5 g (5,5 mMol) Oxalsäure versetzt und unter gutem Rühren wird Luft eingeleitet. Nach 1 h ist die Ausfällung des Cobaltoxalats komplett. Dieses wird abfiltriert. Anschließend wird in einem Dünnschichtverdampfer (Öltemperatur 140 bis 150°C) aus der Lösung das Ethylacetat entfernt. Das als Konzentrat abfließende, geschmolzene Rohprodukt wird in 200 ml 60 - 80°C warmem Wasser dispergiert (emulgiert), um Reste von Lösemittel, Formaldehyd und der Säure aus Reaktionsstufe 1, zu entfernen. Die Emulsion wird unter Rühren langsam abgekühlt, bei 15 bis 5°C kristallisiert das N-Lauroyl-1-propyl-sarkosin. Es wird abgesaugt, mit Wasser nachgewaschen und trockengepreßt.

Ausbeute an N-Lauroyl-1-propyl-sarkosin = 27,0 g ≙ 86 % d.Th. Das trockne Produkt hat einen Fp = 50 bis 51°C.

## Patentansprüche

1. Verfahren zur Herstellung von Acylglycinderivaten der allgemeinen Formel (I) 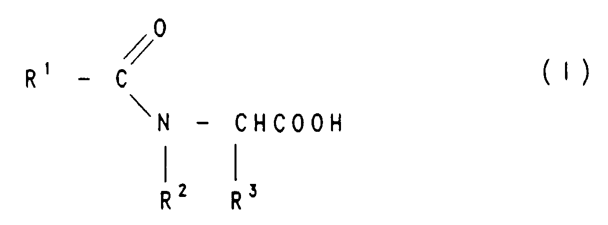 worin
R¹ Wasserstoff, ein gesättigter, geradkettiger, verzweigter oder zyklischer (C₁-C₂₆)Alkylrest, ein ein- oder mehrfach ungesättigter, geradkettiger, verzweigter oder zyklischer (C₂-C₂₄)Alkenylrest, ein (C₆-C₁₈)Arylrest, ein (C₁-C₁₀)Alkyl-(C₆-C₁₈)Arylrest oder ein gegebenenfalls mehrfach ungesättigter (C₂-C₁₀)Alkenyl-(C₆-C₁₈)Arylrest,
R² Wasserstoff, ein gesättigter, geradkettiger, verzweigter oder zyklischer (C₁-C₂₆)Alkylrest, ein ein- oder mehrfach ungesättigter, geradkettiger, verzweigter oder zyklischer (C₂-C₂₃)Alkenylrest, ein (C₆-C₁₈)Arylrest, ein (C₁-C₁₀)Alkyl-(C₆-C₁₈)-Arylrest oder ein gegebenenfalls mehrfach ungesättiger (C₂-C₁₀)Alkenyl-(C₆-C₁₈)Arylrest
und
R³ Wasserstoff, ein gesättigter, geradkettiger, verzweigter oder zyklischer (C₁-C₁₀)Alkylrest, ein ein- oder mehrfach ungesättigter, gradkettiger, verzweigter oder zyklischer (C₂-C₁₀)Alkenylrest, ein (C₆-6₁₈)Arylrest, ein (C₁-C₁₀)Alkyl-(C₆-C₁₈)Arylrest oder ein gegebenenfalls mehrfach ungesättigter (C₂-C₁₀)Alkenyl-(C₆-C₁₈)-Arylrest bedeuten,
dadurch gekennzeichnet, daß man ein Carbonsäureamid der allgemeinen Formel (II) 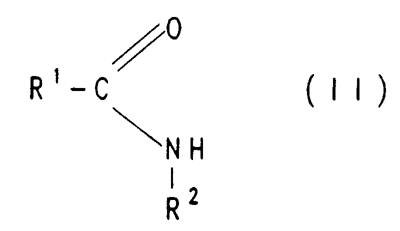 worin R¹ und R² die oben angegebene Bedeutung besitzen mit einem Aldehyd der Formel R³CHO in Gegenwart eines Lösungsmittels und einer Säure zu einem Acylaminomethylol der Formel (III) 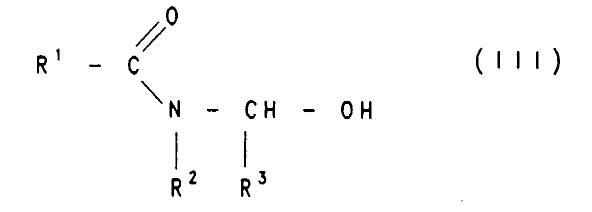 umsetzt und dieses anschließend nach Zusatz eines Cobaltcarbonylkatalysators bei einer Temperatur von 20 bis 150°C und einem CO-Druck von 1 bis 150 bar carbonyliert.

2. Verfahren nach Anspruch 1, dadurch gekennzeichnet, daß als Verbindung der Formel II die Amide der natürlichen Fettsäuren eingesetzt werden.

3. Verfahren nach Anspruch 1, dadurch gekennzeichnet, daß R² für Wasserstoff oder (C₁-C₄)Alkyl, insbesondere Methyl steht.

4. Verfahren nach Anspruch 1, dadurch gekennzeichnet, daß als Verbindung der Formel (II) Octansäureamid, 2-Ethylhexansäureamid, Decansäureamid, Laurinsäureamid, Palmitinsäureamid, Stearinsäureamid, Laurinsäure-N-methylamid, Palmitinsäure-N-methylamid, Stearinsäure-N-methylamid oder Ölsäure-N-methylamid eingesetzt werden.

5. Verfahren nach mindestens einem der Ansprüche 1 bis 4, dadurch gekennzeichnet, daß die Verbindungen der Formel (II) als Gemische, wie sie aus Naturprodukten erhältlich sind, eingesetzt werden.

6. Verfahren nach mindestens einem der Ansprüche 1 bis 5, dadurch gekennzeichnet, daß Formaldehyd in Form von Paraformaldehyd, eingesetzt wird.

7. Verfahren nach mindestens einem der Ansprüche 1 bis 6, dadurch gekennzeichnet, daß der Aldehyd in Mengen von 70 bis 200 Mol-%, insbesondere 100 bis 140 Mol-%, bevorzugt 100 bis 120 Mol-% bezogen auf das Carbonsäureamid eingesetzt wird.

8. Verfahren nach mindestens einem der Ansprüche 1 bis 7, dadurch gekennzeichnet, daß als Säure ein Ionenaustauscherharz oder eine organische oder anorganische Säure, insbesondere Toluolsulfonsäure, Hexafluorpropansulfonsäure, Trifluoressigsäure, Schwefelsäure oder Phosphorsäure, bevorzugt Schwefelsäure eingesetzt wird.

9. Verfahren nach mindestens einem der Ansprüche 1 bis 8, dadurch gekennzeichnet, daß die Säure in einer Menge von 0,2 bis 5 Mol-%, insbesondere 0,5 bis 4 Mol-%, bevorzugt 1,0 bis 2,5 Mol-%, bezogen auf Amid eingesetzt wird.

10. Verfahren nach mindestens einem der Ansprüche 1 bis 9, dadurch gekennzeichnet, daß als Lösungsmittel ein dipolar aprotisches Lösungsmittel, insbesondere Tetrahydrofuran, Glykoldimethylether, Diglykoldimethylether, Dimethylformamid, Dimethylacetamid, bevorzugt Tetrahydrofuran, Glykoldimethylether oder Methyl-t-butylether eingesetzt wird.

11. Verfahren nach mindestens einem der Ansprüche 1 bis 10, dadurch gekennzeichnet, daß die Umsetzung des Amids mit dem Aldehyd bei Temperaturen von 65° bis 120°C durchgeführt wird.

12. Verfahren nach mindestens einem der Ansprüche 1 bis 11, dadurch gekennzeichnet, daß die Carbonylierung bei einer Temperatur von 25 bis 100°C, insbesondere 30 bis 70°C, durchgeführt wird.

13. Verfahren nach mindestens einem der Ansprüche 1 bis 12, dadurch gekennzeichnet, daß die Carbonylierung bei einem CO-Druck von 1 bis 50 bar, bevorzugt 3 bis 20 bar, durchgeführt wird.

14. Verfahren nach mindestens einem der Ansprüche 1 bis 13, dadurch gekennzeichnet, daß für die Carbonylierung reines Kohlenmonoxid eingesetzt wird.

15. Verfahren nach mindestens einem der Ansprüche 1 bis 13, dadurch gekennzeichnet, daß für die Carbonylierung ein Gemisch aus Kohlenmonoxid und Wasserstoff eingesetzt wird.

16. Verfahren nach mindestens einem der Ansprüche 1 bis 15, dadurch gekennzeichnet, daß als Cobaltcarbonyl Co₂(CO)₈ eingesetzt wird.

17. Verfahren nach mindestens einem der Ansprüche 1 bis 16, dadurch gekennzeichnet, daß das Cobaltcarbonyl in einer Menge von 0,1 bis 5,0 Mol-%, insbesondere 0,6 bis 2,0 Mol-% Cobalt, bezogen auf das Carbonsäureamid, eingesetzt wird.

## Claims

1. A process for the preparation of an acylglycine derivative of the formula (I) 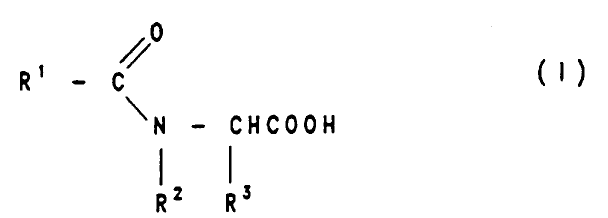 in which
R¹ is hydrogen, a saturated, straight-chain, branched or cyclic (C₁-C₂₆)alkyl radical, a mono- or polyunsaturated, straight-chain, branched or cyclic (C₂-C₂₄) alkenyl radical, a (C₆-C₁₈)aryl radical, a (C₁-C₁₀)alkyl- (C₆-C₁₈)aryl radical or an optionally polyunsaturated (C₂-C₁₀) alkenyl-(C₆-C₁₈)aryl radical,
R² is hydrogen, a saturated, straight-chain, branched or cyclic (C₁-C₂₆)alkyl radical, a mono- or polyunsaturated, straight-chain, branched or cyclic (C₂-C₂₃)alkenyl radical, a (C₆-C₁₈)aryl radical, a (C₁-C₁₀)alkyl- (C₆-C₁₈)aryl radical or an optionally polyunsaturated (C₂-C₁₀) alkenyl-(C₆-C₁₈)aryl radical
and
R³ is hydrogen, a saturated, straight-chain, branched or cyclic (C₁-C₁₀)alkyl radical, a mono- or polyunsaturated, straight-chain, branched or cyclic (C₂-C₁₀)alkenyl radical, a (C₆-C₁₈)aryl radical, a (C₁-C₁₀)alkyl-(C₆-C₁₈)aryl radical or an optionally polyunsaturated (C₂-C₁₀)alkenyl-(C₆-C₁₈)aryl radical,
which comprises reacting a carboxylic acid amide of the formula (II) 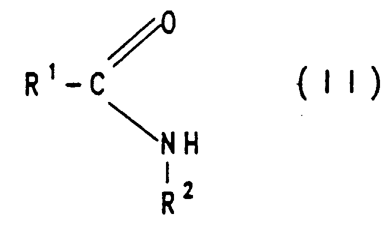 in which R¹ and R² have the abovementioned meaning, with an aldehyde of the formula R³CHO in the presence of a solvent and an acid to give an acylaminomethylol of the formula (III) 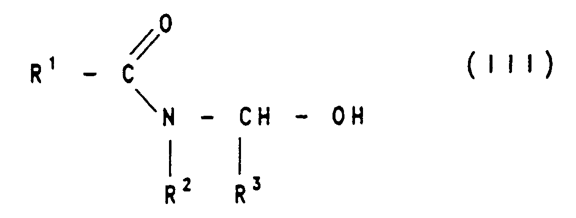 and then carbonylating this, after addition of a cobalt carbonyl catalyst, at a temperature of 20 to 150°C under a CO pressure of 1 to 150 bar.

2. The process as claimed in claim 1, wherein the amide of a naturally occurring fatty acid is employed as the compound of the formula II.

3. The process as claimed in claim 1, wherein R² is hydrogen or (C₁-C₄)alkyl, in particular methyl.

4. The process as claimed in claim 1, wherein octanoic acid amide, 2-ethylhexanoic acid amide, decanoic acid amide, lauric acid amide, palmitic acid amide, stearic acid amide, lauric acid N-methylamide, palmitic acid N-methylamide, stearic acid N-methylamide or oleic acid N-methylamide is employed as the compound of the formula (II).

5. The process as claimed in at least one of claims 1 to 4, wherein the compound of the formula (II) is employed as a mixture such as is obtainable from natural products.

6. The process as claimed in at least one of claims 1 to 5, wherein formaldehyde is employed in the form of paraformaldehyde.

7. The process as claimed in at least one of claims 1 to 6, wherein the aldehyde is employed in an amount of 70 to 200 mol%, in particular 100 to 140 mol%, preferably 100 to 120 mol%, based on the carboxylic acid amide.

8. The process as claimed in at least one of claims 1 to 7, wherein an ion exchanger resin or an organic or inorganic acid, in particular toluenesulfonic acid, hexafluoropropanesulfonic acid, trifluoroacetic acid, sulfuric acid or phosphoric acid, preferably sulfuric acid, is employed as the acid.

9. The process as claimed in at least one of claims 1 to 8, wherein the acid is employed in an amount of 0.2 to 5 mol%, in particular 0.5 to 4 mol%, preferably 1.0 to 2.5 mol%, based on the amide.

10. The process as claimed in at least one of claims 1 to 9, wherein a dipolar aprotic solvent, in particular tetrahydrofuran, glycol dimethyl ether, diglycol dimethyl ether, dimethylformamide or dimethylacetamide, preferably tetrahydrofuran, glycol dimethyl ether or methyl t-butyl ether, is employed as solvent.

11. The process as claimed in at least one of claims 1 to 10, wherein the reaction of the amide with the aldehyde is carried out at a temperature of 65° to 120°C.

12. The process as claimed in at least one of claims 1 to 11, wherein the carbonylation is carried out at a temperature of 25 to 100°C, in particular 30 to 70°C.

13. The process as claimed in at least one of claims 1 to 12, wherein the carbonylation is carried out under a CO pressure of 1 to 50 bar, preferably 3 to 20 bar.

14. The process as claimed in at least one of claims 1 to 13, wherein pure carbon monoxide is employed for the carbonylation.

15. The process as claimed in at least one of claims 1 to 13, wherein a mixture of carbon monoxide and hydrogen is employed for the carbonylation.

16. The process as claimed in at least one of claims 1 to 15, wherein Co₂(CO)₈ is employed as cobalt carbonyl.

17. The process as claimed in at least one of claims 1 to 16, wherein the cobalt carbonyl is employed in an amount of 0.1 to 5.0 mol%, in particular 0.6 to 2.0 mol% of cobalt, based on the carboxylic acid amide.

## Revendications

1. Procédé pour préparer des dérivés d'acylglycine de formule générale (I) 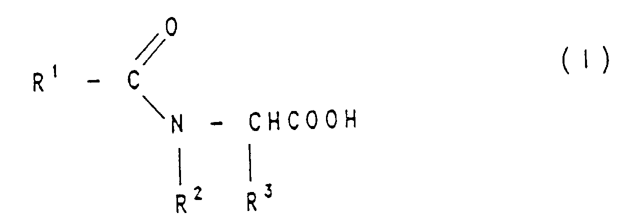 dans laquelle
R¹ est un atome d'hydrogène, un groupe alkyle en C₁-C₂₆ saturé, à chaîne droite, à chaîne ramifiée ou cyclique, un groupe alcényle en C₂-C₂₄ une ou plusieurs fois insaturé, à chaîne droite, ramifiée ou cyclique, un groupe aryle en C₆-C₁₈, un groupe (alkyle en C₁-C₁₀)(aryle en C₆-C₁₈) ou un groupe (alcényle en C₂-C₁₀)(aryle en C₆-C₁₈) éventuellement plusieurs fois insaturé,
R² est un atome d'hydrogène, un groupe alkyle en C₁-C₂₆ saturé, à chaîne droite, à chaîne ramifiée ou cyclique, un groupe alcényle en C₂-C₂₃ une ou plusieurs fois insaturé, à chaîne droite, ramifiée ou cyclique, un groupe aryle en C₆-C₁₈, un groupe (alkyle en C₁-C₁₀)(aryle en C₆-C₁₈) ou un groupe (alcényle en C₂-C₁₀)( aryle en C₆-C₁₈) éventuellement plusieurs fois insaturé,
et
R³ est un atome d'hydrogène, un groupe alkyle en C₁-C₁₀ saturé, à chaîne droite, à chaîne ramifiée ou cyclique, un groupe alcényle en C₂-C₁₀ une ou plusieurs fois insaturé, à chaîne droite, ramifiée ou cyclique, un groupe aryle en C₆-C₁₈, un groupe (alkyle en C₁-C₁₀)(aryle en C₆-C₁₈) ou un groupe (alcényle en C₂-C₁₀)(aryle en C₅-C₁₈) éventuellement plusieurs fois insaturé,
caractérisé en ce qu'on fait réagir un carboxamide de formule générale (II) 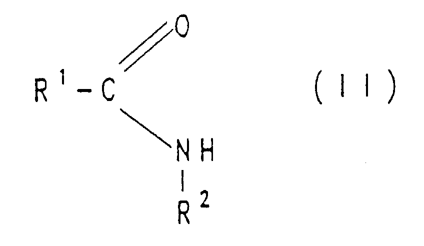 dans laquelle R¹ et R² ont les significations données ci-dessus, avec un aldéhyde de formule R³-CHO en présence d'un solvant et d'un acide, pour obtenir un acylaminométhylol de formule (III) 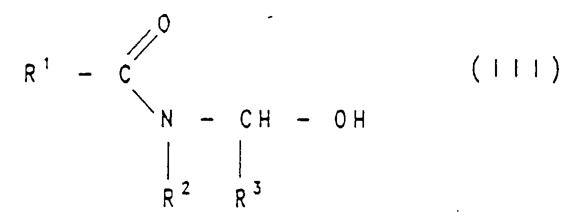 puis on carbonyle ce dernier, après addition d'un catalyseur au cobalt-carbonyle, à une température de 20 à 150°C et sous une pression de CO de 1 à 150 bar.

2. Procédé selon la revendication 1, caractérisé en ce qu'on utilise en tant que composé de formule II les amides des acides gras naturels.

3. Procédé selon la revendication 1, caractérisé en ce que R² est un atome d'hydrogène ou un groupe alkyle en C₁-C₄, en particulier méthyle.

4. Procédé selon la revendication 1, caractérisé en ce qu'on utilise en tant que composé de formule (II) l'octanamide, le 2-éthylhexanamide, le décanamide, le lauramide, le palmitamide, le stéaramide, le N-méthylamide de l'acide laurique, le N-méthylamide de l'acide palmitique, le N-méthylamide de l'acide stéarique ou le N-méthylamide de l'acide oléique.

5. Procédé selon au moins l'une des revendications 1 à 4, caractérisé en ce que les composés de formule (II) sont utilisés sous forme de mélanges tels qu'on peut les obtenir à partir de produits naturels.

6. Procédé selon au moins l'une des revendications 1 à 5, caractérisé en ce que le formaldéhyde est utilisé sous forme de paraformaldéhyde.

7. Procédé selon au moins l'une des revendications 1 à 6, caractérisé en ce que l'aldéhyde est utilisé en des quantités de 70 à 200 % en moles, en particulier de 100 à 140 % en moles et de préférence de 100 à 120 % en moles par rapport au carboxamide.

8. Procédé selon au moins l'une des revendications 1 à 7, caractérisé en ce qu'on utilise en tant qu'acide une résine échangeuse d'ions ou un acide organique ou minéral, en particulier l'acide toluènesulfonique, l'acide hexafluoropropanesulfonique, l'acide trifluoracétique, l'acide sulfurique ou l'acide phosphorique, de préférence l'acide sulfurique.

9. Procédé selon au moins l'une des revendications 1 à 8, caractérisé en ce que l'acide est utilisé en une quantité de 0,2 à 5 % en moles, en particulier de 0,5 à 4 % en moles, de préférence de 1,0 à 2,5 % en moles par rapport à l'amide.

10. Procédé selon au moins l'une des revendications 1 à 9, caractérisé en ce qu'on utilise en tant que solvant un solvant aprotique dipolaire, en particulier le tétrahydrofuranne, l'éther diméthylique du glycol, l'éther diméthylique du diglycol, le diméthylformamide, le diméthylacétamide, de préférence le tétrahydrofuranne, l'éther diméthylique du glycol ou le méthyl-tert-butyléther.

11. Procédé selon au moins l'une des revendications 1 à 10, caractérisé en ce que la réaction de l'amide avec l'aldéhyde est mise en oeuvre à des températures de 65 à 120°C.

12. Procédé selon au moins l'une des revendications 1 à 11, caractérisé en ce que la carbonylation est mise en oeuvre à une température de 25 à 100°C, en particulier de 30 à 70°C.

13. Procédé selon au moins l'une des revendications 1 à 12, caractérisé en ce que la carbonylation est mise en oeuvre sous une pression de CO de 1 à 50 bar, de préférence de 3 à 20 bar.

14. Procédé selon au moins l'une des revendications 1 à 13, caractérisé en ce qu'on utilise du monoxyde de carbone pur pour la carbonylation.

15. Procédé selon au moins l'une des revendications 1 à 13, caractérisé en ce qu'on utilise pour la carbonylation un mélange de monoxyde de carbone et d'hydrogène.

16. Procédé selon au moins l'une des revendications 1 à 15, caractérisé en ce qu'on utilise en tant que cobalt-carbonyle du Co₂(CO)₈.

17. Procédé selon au moins l'une des revendications 1 à 16, caractérisé en ce que le cobalt-carbonyle est utilisé en une quantité de 0,1 à 5,0 % en moles, en particulier de 0,6 à 2,0 % en moles de cobalt par rapport au carboxamide.
